# FASCICULE DE BREVET EUROPEEN

(11) **EP 0 937 039 B1**
(45) Date de publication et mention de la délivrance du brevet: **11.12.2002**
(21) Numéro de dépôt: 97943942.9
(22) Date de dépôt: 03.10.1997
(51) Int. Cl.: C07D 201/08

(54) **PREPARATION DE CAPROLACTAME**
VERFAHREN ZUR HERSTELLUNG VON CAPROLACTAM
PREPARATION OF CAPROLACTAM

(30) Priorité: 04.10.1996 FR 9612326
(43) Date de publication de la demande: 25.08.1999
(73) Titulaire: RHODIA POLYAMIDE INTERMEDIATES, 69190 Saint-Fons (FR)
(72) Inventeur: PATOIS, Carl, F-68400 Riedisheim (FR); SPAGNOL, Michel, F-69003 Lyon (FR)
(74) Mandataire: Esson, Jean-Pierre
(86) Numéro de dépôt international: FR9701761
(87) Numéro de publication internationale: WO98015529

(56) Documents cités:
- EP-A- 0 242 505
- EP-A- 0 729 944
- WO-A-94/26688
- WO-A-95/18089
- WO-A-95/18783
- WO-A-97/02228
- DE-A- 3 602 375
- US-A- 4 730 041
- JERRY MARCH: "Advanced Organic Chemistry, Reactions, Mechanisms and Structure, Third Edition pp. 722-723, 798-800", 1985, JOHN WILEY & SONS,

## Description

La présente invention conceme l'aminométhylation de dérivés des acides penténoïques, par réaction avec le monoxyde de carbone, l'hydrogène et l'ammoniac, afin de préparer le caprolactame.

Le caprolactame est le constituant du Nylon 6.

De nombreuses voies d'accès existent pour le caprolactame, notamment à partir de butadiène.

En particulier, il a été envisagé de préparer successivement un pentène-nitrile par hydrocyanation de butadiène, puis un dérivé de l'acide 5-cyanovalérique, en introduisant la fonction carbonyle grâce à une réaction de carbonylation ou une réaction d'hydroformylation, et finalement transformer la fonction nitrile en fonction amine par une réaction d'hydrogénation, afin d'obtenir le caprolactame et/ou l'acide aminocaproïque. Ce procédé souffre d'un grand nombre d'étapes, et requiert l'utilisation successive d'acide cyanhydrique, puis de monoxyde de carbone, ce qui peut nuire à l'intérêt économique d'un tel procédé.

Le brevet EP-A-0 729 944 décrit un procédé de préparation d'epsilon-caprolactame à partir d'acide 5-formylvalérique, de ses esters ou de l'amide correspondant, consistant à mettre en contact l'un de ces substrats avec de l'ammoniac et de l'eau dans des conditions non hydrogénantes, puis à mettre le mélange obtenu en contact avec de l'hydrogène en présence d'ammoniac dans des conditions hydrogénantes avec une teneur en eau supérieure à 10 %, puis à chauffer le mélange réactionnel à 200 - 350°C.

La demande de brevet WO-A-95/18089 décrit la préparation de l'acide 5-formylvalérique et de ses esters, par réaction de l'acide 3-penténoïque ou de l'un de ses esters avec le monoxyde de carbone et l'hydrogène en présence d'un catalyseur en rhodium et d'un ligand phosphite.

La demande de brevet WO-A-94/26688 décrit la préparation d'un aldéhyde linéaire par hydroformylation d'un composé insaturé qui a préalablement été isomérisé afin qu'il comporte une insaturation terminale, en particulier un 4-penténoate d'alkyle, l'hydroformylation consistant à faire réagir le composé à insaturation terminale avec le monoxyde de carbone et l'hydrogène en présence d'un catalyseur tel qu'un complexe platine/phosphine bidentate.

Les brevets US 4,730,041, EP-A-0 242 505 et DE-A-3 602 375 décrivent différentes variantes de préparation d'epsilon-caprolactame par réaction d'un 5-formylvalérate d'alkyle avec un excès d'ammoniac et d'hydrogène, en présence d'un catalyseur d'hydrogénation, sous une pression supérieure à la pression atmosphérique, en phase liquide et à une température supérieure à la température ambiante (40°C à 130°C ou 50°C à 180°C). L'ammoniac et l'hydrogène en excès sont ensuite séparés et le mélange réactionnel ainsi obtenu est chauffé à 150°C - 250°C pour fournir l'epsilon-caprolactame.

La demande de brevet WO-A-97/02228, qui a une date de priorité antérieure et une date de publication postérieure à la date de priorité de la présente demande de brevet, décrit un procédé de séparation de l'acide 5-formylvalérique linéaire d'un mélange contenant aussi des acides 3-formylvalérique et/ou 4-formylvalérique ramifiés. Cette séparation est réalisée par extraction fractionnée avec deux solvants non-miscibles dont l'un est un solvant aqueux et l'autre un solvant organique.

Aucun des procédés décrits dans ces documents de l'art antérieur ne permet de réaliser en une seule étape la préparation d'epsilon-caprolactame à partir d'un dérivé d'acide penténoïque.

Dans le procédé de l'invention, le caprolactame est obtenu directement en une seule étape à partir d'un dérivé d'acide penténoïque, ce qui est beaucoup plus simple que les procédés déjà envisagés à partir de butadiène.

Les dérivés d'acide penténoïque sont par exemple les esters. De tels esters peuvent quant à eux être facilement obtenus à partir de butadiène selon des procédés bien décrits.

Les métaux pouvant catalyser ce type de réaction sont par exemple le cobalt, le palladium, le rhodium ou l'iridium.

La réaction employée ici, qui consiste à transformer un composé C=C en produit CH-CH-CH₂-N, porte le nom d'aminométhylation. L'aminométhylation d'oléfines est une réaction connue, catalysée par une variété de métaux du groupe VIII ; toutefois elle est en général limitée à la synthèse d'aminés secondaires ou tertiaires, parce que l'amine formée par aminométhylation est un meilleur nucléophile que l'amine de départ.
Ainsi, dans le cas de l'utilisation d'ammoniac, la réactivité du produit formé par aminométhylation de l'oléfine avec l'ammoniac réagit plus vite avec une autre oléfine que l'ammoniac.

Nous avons trouvé que cette limitation pouvait être contournée en utilisant la cyclisation intramoléculaire de l'amine en lactame. A titre d'exemple, ce but est atteint en utilisant comme substrat un ester de l'acide penténoïque.

Plus précisément l'invention consiste en un procédé de préparation de caprolactame, caractérisé en ce que ledit caprolactame est réalisé en une seule étape et en réalisant l'aminométhylation de dérivés des acides penténoïques définis ci-dessous, par réaction avec le monoxyde de carbone, l'hydrogène et l'ammoniac, à une température supérieure à 30°C et en présence d'un catalyseur à base d'au moins un métal du groupe VIII de la Classification périodique des Éléments.

Les acides penténoïques concernés par l'invention sont les divers isomères de ce composé, à savoir les acides cis-pent-2-énoïque, trans-pent-2-énoïque, cis-pent-3-énoïque, trans-pent-3-énoïque et pent-4-énoïque.

Les dérivés des acides penténoïques dans le cadre de l'invention sont les composés où le groupement OH de la fonction acide a été remplacé par un groupement potentiellement meilleur nucléofuge, tel que O-R ou S-R, dans lesquels R représente un reste alkyle, cycloalkyle, aralkyle, aryle, silyle. Plus particulièrement, les dérivés utilisés sont les esters d'alcools comportant de 1 à 6 atomes de carbone.

On peut utiliser un mélange quelconque de dérivés des divers isomères penténoïques. Toutefois, on utilisera de préférence des mélanges où les dérivés des acides pent-3-énoïques sont majoritaires. Le mélange de dérivés d'acides penténoïques peut contenir en outre une quantité minoritaire de 4-méthylbutyrolactone et de dérivés d'autres acides carboxyliques à 5 atomes de carbone tel que l'acide valérique, à une teneur de préférence inférieure à 20 % en poids, et encore plus préférentiellement inférieure à 10 % en poids.

La concentration en poids des dérivés d'acides penténoïques peut varier dans de larges limites, selon que l'on opère en présence d'un solvant ou non, et selon le degré d'avancement de la réaction. A titre indicatif, elle est par exemple comprise entre 5 et 99 % en poids.

La réaction est avantageusement conduite en présence d'un solvant. La quantité de solvant mise en oeuvre varie entre 10 et 95 % en poids du milieu réactionnel.

Ce solvant peut être de nature très variée. On peut notamment choisir les alcools, les éthers aliphatiques, les éthers aromatiques, les éthers mixtes, les nitriles, les amides acycliques, les amides cycliques, les hydrocarbures aliphatiques, les hydrocarbures aromatiques, les hydrocarbures aliphatiques chlorés, les hydrocarbures cycloaliphatiques chlorés ou les mélanges de plusieurs de ces solvants.

Dans le cas où l'on utilise un alcool comme solvant ou comme partie d'un mélange de solvants, et que l'on réalise l'aminométhylation d'un ester d'acide penténoïque, l'alcool correspondant au dit ester est préférentiellement utilisé.

A titre d'exemples non limitatifs, les solvants pouvant être utilisés sont l'éthanol, le méthanol, l'isopropanol, le diphényléther, l'éther isopropylique, l'acétonitrile, le diméthylformamide, la N-méthyl-pyrrolidone, le benzène, le toluène, les xylènes, les chlorobenzènes, le dichlorométhane, les dichloroéthanes, l'hexane et le cyclohexane.

Dans le cadre d'une mise en oeuvre industrielle du procédé, des recyclages du catalyseur et des dérivés d'acides penténoïques n'ayant pas réagi peuvent conduire à l'introduction dans le milieu réactionnel de quantités plus ou moins importantes d'autres composés, et en particulier de caprolactame produit. Dans le cadre de l'invention, ces composés seront considérés comme faisant partie intégrante du système solvant.

Le catalyseur est à base d'au moins un métal du groupe VIII. Comme source de tels catalyseurs peuvent être utilisés les éléments métalliques, divers sels ou complexes organométalliques tels que les chlorures, les iodures, les bromures, les métaux carbonyles, les carboxylates, ceux-ci étant mentionnés à titre d'exemples non limitatifs.

De préférence, le catalyseur est à base d'au moins un des métaux pris parmi le rhodium, l'iridium, le ruthénium, le fer, ou le cobalt. Optionnellement, le catalyseur est associé à des promoteurs, tels que des dérivés du phosphore et/ou des sels Qⁿ⁺ Xₙ où n représente 1 ou 2, Q représentant un élément alcalin (n=1) ou alcalino-terreux (n=2), et X un halogène.

De manière préférée, Q est choisi parmi le lithium, le sodium, le potassium, le magnésium et le calcium, X est choisi parmi le fluor, le chlore, le brome et l'iode.

Les dérivés du phosphore sont du type R¹R²R³P, où R¹, R², R³ sont égaux ou différents et représentent un reste alkyle ou aralkyle ou cycloalkyle comportant de 1 à 20 atomes de carbones, pouvant comporter en outre un ou plusieurs atomes pris parmi l'azote, l'oxygène, le soufre le phosphore et le fer, et pouvant former entre eux un ou plusieurs cycles. A titre d'exemples illustratifs non limitatifs de tels promoteurs, conviennent la triphénylphosphine, la tricyclohexylphosphine, les phosphanorbomadiènes, les phosphanorbomènes, les phosphanorbomanes, le 1,2-bis(diphényl-phosphino)-éthane, le 1,2-bis(diphénylphosphinométhyl)-cyclobutane, le 1,3-bis(diphénylphosphino)-propane, le 1,4-bis(diphénylphosphino)-butane, le 1,1'-bis(diphénylphosphino)-ferrocène.

La quantité de catalyseur à mettre en oeuvre peut varier dans de larges limites. En général, une quantité, exprimée en nombre total de moles de métaux utilisés comme catalyseurs par litre de mélange réactionnel, comprise entre 10⁻⁵ et 10⁻¹ conduit à des résultats satisfaisants. Des quantités inférieures peuvent être utilisées, mais on observe toutefois que la vitesse de réaction est faible. Des quantités supérieures n'ont d'inconvénient qu'au plan économique. De préférence, cette quantité est comprise entre 10⁻⁴ et 5.10⁻² mole par litre de mélange réactionnel.

La réaction d'aminométhylation peut être conduite à une température généralement située entre 60°C et 230°C, et de préférence entre 80°C et 200°C.

La pression partielle de monoxyde de carbone, mesurée à 25°C, est comprise entre 0,5 et 300 bars. Elle est de préférence comprise entre 2 et 200 bars, et encore plus préférentiellement entre 5 et 150 bars.

La pression partielle d'hydrogène, mesurée à 25°C, est comprise entre 0,5 et 200 bars. Elle est de préférence comprise entre 1 et 150 bars, et encore plus préférentiellement entre 2 et 100 bars.

Le rapport molaire hydrogène / monoxyde de carbone est compris entre 10/1 et 1/10, de préférence entre 5/1 et 1/2.

La quantité d'ammoniac mise en jeu peut varier dans de larges limites. Toutefois, il est préférable d'utiliser un excès par rapport au substrat. Le rapport molaire ammoniac / substrat est compris entre 1/1 et 100/1 et de préférence entre 1,1/1 et 50/1.

Le procédé de l'invention peut être mis en oeuvre de manière continue ou discontinue. Selon la mise en oeuvre choisie, il y aura donc lieu d'adapter les différentes conditions opératoires définies précédemment.

L'exemple qui suit illustre l'invention.

Dans un autoclave de 125 ml en Hastelloy® B2, sont introduits successivement 88,6 mg de Rh₆(CO)₁₆, 1,11 g de penténoate de méthyle, 20 ml de méthanol ; l'autoclave est fermé et 4,58 g de NH₃ sont introduits au moyen d'un sas. L'autoclave est alors pressurisé par 40 bar de CO et 80 bar d'hydrogène à 20°C. L'agitation par secousses est démarrée et la température de l'autoclave est portée à 150°C ; la pression totale est alors de 163 bar. Au bout de 195 min la pression dans l'autoclave a diminué de 2 bar ; le chauffage est alors arrêté. Après refroidissement, l'autoclave est dégazé, puis ouvert ; la masse réactionnelle vert kaki est analysée en Chromatographie en Phase Gazeuse (CPG). Le rendement de 33 % en caprolactame est confirmé par un couplage CPG/SM (spectrométrie de masse).

## Revendications

1. Procédé de préparation de caprolactame, **caractérisé en ce que** le dit caprolactame est obtenu directement en une seule étape et en réalisant l'aminométhylation de dérivés des acides penténoïques comprenant un groupement OH d'une fonction acide remplacé par un groupement plus nucléofuge que ledit groupement OH par réaction avec le monoxyde de carbone, l'hydrogène et l'ammoniac, à une température supérieure à 30°C et en présence d'un catalyseur à base d'au moins un métal du groupe VIII de la Classification périodique des Eléments.

2. Procédé selon la revendication 1, **caractérisé en ce que** le groupement plus nucléofuge est choisi dans le groupe comprenant les groupements O-R et S-R dans lesquels R représente un reste alkyle, cycloalkyle, aralkyle, aryle, silyle.

3. Procédé selon la revendication 1 ou 2, **caractérisé en ce que** l'on opère en présence d'un solvant.

4. Procédé selon l'une quelconque des revendications 1 à 3, **caractérisé en ce que** la quantité totale de catalyseur est comprise entre 10⁻⁵ et 10⁻¹ mole par litre de mélange réactionnel, et de préférence comprise entre 10⁻⁴ et 5.10⁻² mole par litre de mélange réactionnel.

5. Procédé selon l'une quelconque des revendications 1 à 4, **caractérisé en ce que** la température est comprise entre 60°C et 230°C, et de préférence entre 80°C et 200°C.

6. Procédé selon l'une quelconque des revendications 1 à 5, **caractérisé en ce que** le catalyseur est à base d'au moins un des métaux pris parmi le rhodium, l'iridium, le ruthénium, le fer, ou le cobalt.

7. Procédé selon l'une quelconque des revendications 1 à 6, **caractérisé en ce que** le rapport molaire ammoniac / substrat est compris entre 1/1 et 100/1, et de préférence entre 1,1/1 et 50/1.

8. Procédé selon l'une quelconque des revendications 1 à 7, **caractérisé en ce que** la pression partielle de monoxyde de carbone, mesurée à 25°C, est comprise entre 0,5 et 300 bars, de préférence comprise entre 2 et 200 bars, et encore plus préférentiellement entre 5 et 150 bars.

9. Procédé selon l'une quelconque des revendications 1 à 8, **caractérisé en ce que** la pression partielle d'hydrogène, mesurée à 25°C, est comprise entre 0,5 et 200 bars, de préférence comprise entre 2 et 150 bars, et encore plus préférentiellement entre 5 et 100 bars.

10. Procédé selon l'une quelconque des revendications 1 à 9, **caractérisé en ce que** le rapport molaire hydrogène / monoxyde de carbone est compris entre 10/1 et 1/10, de préférence entre 5/1 et 1/2.

11. Procédé selon l'une quelconque des revendications 1 à 10, **caractérisé en ce que** le dérivé d'acide penténoïque est un ester, de préférence un ester méthylique ou éthylique.

## Claims

1. Process for the preparation of caprolactam, **characterized in that** the said caprolactam is obtained directly in a single stage and by carrying out the aminomethylation of pentenoic acid derivatives, comprising an OH group of an acid functional group replaced by a more nucleofugic group than the said OH group, by reaction with carbon monoxide, hydrogen and ammonia, at a temperature greater than 30°C and in the presence of a catalyst based on at least one metal from group VIII of the Periodic Classification of the Elements.

2. Process according to Claim 1, **characterized in that** the more nucleofugic group is chosen from the group consisting of O-R and S-R groups in which R represents an alkyl, cycloalkyl, aralkyl, aryl or silyl residue.

3. Process according to claim 1 or 2, **characterized in that** the reaction is carried out in the presence of a solvent.

4. Process according to any one of claims 1 to 3, **characterized in that** the total amount of catalyst is between 10⁻⁵ and 10⁻¹ mol per litre of reaction mixture and preferably between 10⁻⁴ and 5 x 10⁻² mol per litre of reaction mixture.

5. Process according to any one of claims 1 to 4, **characterized in that** the temperature is between 60°C and 230°C and preferably between 80°C and 200°C.

6. Process according to any one of claims 1 to 5, **characterized in that** the catalyst is based on at least one of the metals taken from rhodium, iridium, ruthenium, iron or cobalt.

7. Process according to any one of claims 1 to 6, **characterized in that** the ammonia/substrate molar ratio is between 1/1 and 100/1 and preferably between 1.1/1 and 50/1.

8. Process according to any one of claims 1 to 7, **characterized in that** the carbon monoxide partial pressure, measured at 25°C, is between 0.5 and 300 bar, preferably between 2 and 200 bar and more preferably still between 5 and 150 bar.

9. Process according to any one of claims 1 to 8, **characterized in that** the hydrogen partial pressure, measured at 25°C, is between 0.5 and 200 bar, preferably between 2 and 150 bar and more preferably still between 5 and 100 bar.

10. Process according to any one of claims 1 to 9, **characterized in that** the hydrogen/carbon monoxide molar ratio is between 10/1 and 1/10, preferably between 5/1 and 1/2.

11. Process according to any one of claims 1 to 10, **characterized in that** the pentenoic acid derivative is an ester, preferably a methyl or ethyl ester.

## Patentansprüche

1. Verfahren zur Herstellung von Caprolactam, **dadurch gekennzeichnet, daß** das genannte Caprolactam direkt in nur einer einzigen Stufe erhalten wird, indem man die Aminomethylierung von Pentensäure-Derivaten, bei denen die OH-Gruppe einer Säurefunktion durch eine kernflüchtigere Gruppe als die genannte OH-Gruppe ersetzt ist, durch Reaktion mit Kohlenmonoxid, Wasserstoff und Ammoniak bei einer Temperatur von über 30 °C und in Anwesenheit eines Katalysators auf der Basis von mindestens einem Metall der Gruppe VIII des Periodensystems der Elemente realisiert.

2. Verfahren nach Anspruch 1, **dadurch gekennzeichnet, daß** die kernflüchtigere Gruppe aus der die Gruppen O-R und S-R umfassenden Gruppe gewählt wird, worin R einen Rest Alkyl, Cycloalkyl, Aralkyl, Aryl, silyl darstellt.

3. Verfahren nach Anspruch 1 oder 2, **dadurch gekennzeichnet, daß** man in Anwesenheit eines Lösungsmittels arbeitet.

4. Verfahren nach irgendeinem der Ansprüche 1 bis 3, **dadurch gekennzeichnet, daß** die Gesamtmenge an Katalysator zwischen 10⁻⁵ und 10⁻¹ Mol pro Liter Reaktionsmischung und vorzugsweise zwischen 10⁻⁴ und 5.10⁻² Mol pro Liter Reaktionsmischung beträgt.

5. Verfahren nach irgendeinem der Ansprüche 1 bis 4, **dadurch gekennzeichnet, daß** die Temperatur zwischen 60 °C und 230 °C, vorzugsweise zwischen 80 °C und 200 °C beträgt.

6. Verfahren nach irgendeinem der Ansprüche 1 bis 5, **dadurch gekennzeichnet, daß** der Katalysator auf der Basis von mindestens einem der Metalle beruht, die unter Rhodium, Iridium, Ruthenium, Eisen oder Cobalt ausgewählt sind.

7. Verfahren nach irgendeinem der Ansprüche 1 bis 6, **dadurch gekennzeichnet, daß** das molare Verhältnis Ammoniak/Substrat zwischen 1/1 und 100/1, vorzugsweise zwischen 1,1/1 und 50/1 beträgt.

8. Verfahren nach irgendeinem der Ansprüche 1 bis 7, **dadurch gekennzeichnet, daß** der partielle Druck des Kohlenmonoxids, gemessen bei 25 °C, zwischen 0,5 bar und 300 bar, vorzugsweise zwischen 2 bar und 200 bar und noch mehr bevorzugt zwischen 5 bar und 150 bar beträgt.

9. Verfahren nach irgendeinem der Ansprüche 1 bis 8, **dadurch gekennzeichnet, daß** der partielle Druck des Wasserstoffs, gemessen bei 25 °C, zwischen 0,5 bar und 200 bar, vorzugsweise zwischen 2 bar und 150 bar und noch mehr bevorzugt zwischen 5 bar und 100 bar beträgt.

10. Verfahren nach irgendeinem der Ansprüche 1 bis 9, **dadurch gekennzeichnet, daß** das molare Verhältnis Wasserstoff/Kohlenmonoxic zwischen 10/1 und 1/10, vorzugsweise zwischen 5/1 und 1/2 beträgt.

11. Verfahren nach irgendeinem der Ansprüche 1 bis 10, **dadurch gekennzeichnet, daß** das Pentensäure-Derivat ein Ester, vorzugsweise ein Methylester oder Ethylester ist.
